# EUROPEAN PATENT SPECIFICATION

(11) **EP 1 725 215 B1**
(45) Date of publication and mention of the grant of the patent: **25.07.2007**
(21) Application number: 05717901.2
(22) Date of filing: 03.03.2005
(51) Int. Cl.: A61K 9/127, B01J 13/02

(54) **METHOD AND APPARATUS FOR PRODUCING CARRIER COMPLEXES**
VERFAHREN UND GERÄT ZUR HERSTELLUNG VON TRÄGERKOMPLEXEN
PROCEDE ET APPAREIL DE PRODUCTION DE COMPLEXES D'EXCIPIENT

(30) Priority: 05.03.2004 GB 0404993
(43) Date of publication of application: 29.11.2006
(73) Proprietor: Liverpool John Moores University, Liverpool L3 3AF (GB)
(72) Inventor: MOZAFARI, Mohammad, Reza, Liverpool L3 3AF (GB)
(74) Representative: McCall, John Douglas
(86) International application number: PCT/GB2005/000825
(87) International publication number: WO 2005/084641

(56) References cited:
- EP-A- 0 211 647
- EP-A- 0 753 340
- EP-A- 0 970 982
- DE-A1- 10 061 656
- DE-A1- 19 924 643
- JP-A- 63 119 848

## Description

The present invention relates to a method of producing a carrier complex of an active ingredient disposed within a carrier material and additionally relates to an apparatus for producing such carrier complexes.

To improve methods of drug administration, increasing efforts have been devoted to designing effective delivery systems. A drug delivery system can be defined as the technology necessary for optimising the therapeutic efficiency of a drug. Controlled drug delivery technology represents one of the frontier areas of science, involving multidisciplinary approaches, contributing to human and animal health. Controlled drug delivery systems (referred to as carrier systems) can be broadly categorised as polymer, lipid and surfactant-based systems and combinations of them.

Typical examples for drug delivery systems are microspheres (Shen, C., et al., (1994) J. Controlled Release 32: 139-146)*,* nanospheres (polymer-based systems) (Ravi Kumar, M.N.V., et al., (2004) Biomaterials 25: 1771-1777*),* liposomes (Abra, R.M, et al., (2002) J. Liposome Res. 12 (1 & 2): 1-3*),* vesicular phospholipid gels *(*Kaiser, N., et al., (2003) Int. J. Pharmaceutics 256: 123-131*),* archaeosomes (lipid-based systems) *(*Patel, G.B., et al., (2002) J. Liposome Res. 12: 353-372*),* and niosomes (surfactant-based systems) *(*Nasseri, B. and Florence, A.T (2003) Int. J. Pharmaceutics 54: 11-16). Examples of combined carrier systems are micelles (Torchilin, V. (1998) J. Microencapsulation 15: 1-19*)* and nanoparticles *(*Huang, G., et al., (2004) J. Controlled Release 94: 303-311*)* that can be prepared from polymer-lipid conjugates and lipid-surfactant mixtures respectively.

There are numerous lab-scale and a few large-scale techniques for the preparation of the above-mentioned carrier systems. Examples for these techniques include lipid hydration method (for preparation of niosomes) *(*Pillai, G.K. and Salim, M.L.D. (1999) Int. J. Pharmaceutics 193: 123-127*),* high-pressure homogenisation (for preparation of vesicular phospholipid gels), precipitation polymerisation (for preparation of nanoparticles) *(*Pelton, R.H. and Chibante, P. (1986) Colloids and Surfaces 20: 247-256*),* emulsification-diffusion method (for preparation of nanoparticles) *(*Quintanar-Guerrero, D., et al., (1996) Int. J. Pharmaceutics 143: 133-141) and emulsion-solvent evaporation technique (for preparation of microspheres) *(*Kim, C.K., et al., (1990) Arc. Pharmacal Res. 13: 293-297*).*

However, most of these techniques for producing the carrier systems are not suitable for the encapsulation of sensitive substances because of their exposure either to mechanical stresses (e.g. sonication, high-shear homogenisation, high pressures) or potentially harmful chemicals (e.g. volatile organic solvents, low/high values of pH) during the preparation. The majority of these preparation techniques involve the application of harmful volatile organic solvents (e.g. chloroform, ether, methanol, acetone) to dissolve or solubilise the ingredients. These solvents not only affect the chemical structure of the entrapped substance but also will remain in the final formulation and contribute to toxicity and influence the stability of the carrier systems. In general, residual solvents in pharmaceuticals are known as organic volatile impurities and have no therapeutic benefits, but may be hazardous to human and animal health as well as the environment *(*Dwivedi, A.M. (2002) Pharma. Tech. Europe 14: 26-28)*.* In addition to the above mentioned disadvantages, application of volatile organic solvents necessitates performance of two additional steps in the preparation of the carrier systems: i) removal of these solvents, and ii) assessment of the level of residual organic solvents in the formulations. Hence, avoiding the utilisation of these solvents and harmful procedures will bring down the toxicity as well as the time and cost of preparation of the carrier systems. Whilst methods for producing carrier systems that do not utilise harmful solvents have been previously described (Mozafari, M.R. et al., (2002) Cell. Molec. Biol. Letts. (7) 923-927), the lipids forming the encapsulate are hydrated separately for a prolonged period prior to being mixed together at a high temperature prior to incubation with nucleic acids. Therefore, such methods are not only laborious, but also time consuming. The cost of preparing pharmaceutically active molecules within carrier systems can be further brought down by reducing the number of steps and time it takes to produce these pharmaceuticals into a formulation that is suitable for human or animal use.

It is therefore an object of the present invention to overcome one or more problems associated with prior art methods of producing carrier complexes. It is also an object of the present invention to provide a method of producing a carrier complex which is capable of producing a vast number of polymer, lipid and surfactant-based carrier complexes in a single step and in a single piece of equipment or vessel and without involving harmful substances or procedures. Furthermore, it would be preferable that the method was economical with a simple protocol and that the resultant carrier complex has a superior monodispersity and storage stability. It would be most desirable if the method could be adapted from small to industrial scales.

In accordance with the present invention, there is provided a method of producing a carrier complex of an active ingredient disposed within a carrier material, the method comprising the steps of;
(a) providing a complexation zone supplied with an aqueous medium containing the carrier material and a non-volatile water soluble organic solvent;
(b) simultaneously stirring, heating, and passing an inert gas through the aqueous medium;
(c) adding the active ingredient to the aqueous medium and maintaining the complexation zone under conditions of temperature effective to facilitate complexation of the active ingredient by the carrier material; and
(d) recovering from the complexation zone a carrier complex of the active ingredient.

The term "carrier complex" relates to an active ingredient which is surrounded by, bonded to or encapsulated by a carrier material such that the complex remains relatively stable in a number of *in vivo* and *in vitro* cellular environments. Such an environment may be the cytosol or bodily fluids. Whilst such a complex may be designed so that it does not produce an antigenic effect for the delivery of an active ingredient (for example the delivery of DNA to a cell), it may also be designed such that it initiates an antigenic effect on the active ingredient (for example for use as a vaccine). It follows that the term "complexation" is the process of an active ingredient being surrounded by, bonded to or encapsulated by a carrier material.

The present invention therefore provides for a method which does not require volatile organic solvents to be used in the production of carrier complexes and the water soluble organic solvents also serve as dispersants and prevent coagulation/aggregation or sedimentation of the carrier complexes thereby enhancing the stability of the preparations. The water soluble organic solvents also improve the stability of the carrier complexes against freezing, thawing, etc. The inert gas which is used as a means for enhancing the stirring and hydration of the components also functions to prevent oxidation of the components enhancing the stability and shelf life of the products. Furthermore, the method does not require the carrier materials to be hydrated separately prior to heating and this further allows the method to be more efficient.

Step (c) of the method may be incorporated into step (a) and therefore the active ingredient (such as a pharmaceutical compound) can be incorporated in the initial mixture of the aqueous medium. Alternatively, step (c) may be incorporated into step (b). Furthermore, step (c) may be subjected to simultaneous stirring and the passing of inert gas through the aqueous medium. The aqueous medium of step (b) and/or (c) may be heated to a temperature in the range of 40°C to 120°C. Preferably, the aqueous medium of step (b) is heated to a temperature in the range of 70°C to 120°C. Step (c) of the method may be further subjected to a temperature that is less than the temperature of the aqueous medium in step (b) and this may be necessary should the active ingredient be sensitive to high temperatures (such as nucleic acids). Preferably, should step (c) of the method may be further subjected to a temperature that is less than the temperature of the aqueous medium in step (b), the temperature is in the range of 40°C to 60 °C. If sterols are used, it is preferred to prepare the carrier complexes at 120°C., however in the absence of sterols, a temperature of 70°C to 90°C should be sufficient. It will be apparent to one skilled in the art that commonly, steps (a) to (c) will be performed simultaneously, provided that the active ingredient is not sensitive to heat. Should the active ingredient be sensitive to heat, then only steps (a) and (b) will be performed simultaneously.

Preferably, the inert gas is nitrogen. The inert gas may be at least partially recycled in order that the method is run efficiently with the minimum wastage. It will be evident to one skilled in the art that the inert gas may be completely recycled or a depleted stream of inert gas could be mixed with a fresh stream.

Preferably, the method has a duration time of up to 2 hours, although it is envisaged that most carrier complexes will be produced to a satisfactory quantity and quality within a 30 minute period.

The carrier complex may comprise a microsphere, a nanosphere, a liposome, a vesicular phospholipid gel, an archaeosome, a niosome, a micelle or a nanoparticle.

The carrier material used in the method may be selected from one or more of the following: a copolymer, a synthetic polymer-lipid conjugate, a phospholipid or a non-ionic surfactant. Examples of carrier material include copolymers such as poly(DL-lactic acid)-poly(ethylene glycol) (PDLLA-PEG), poly(N-isopropylacrylamide)-b-poly(ethylene glycol) (PNIPAAm-b-PEG), and dextran-hydroxyethyl methacrylate-poly(ethylene glycol) (dexHEMA-PEG), synthetic polymer-lipid conjugates such as poly(ethylene glycol)-phosphatidyl ethanolamine (PEG-PE), poly(ethylene glycol)-distearoyl phosphatidyl ethanolamine (PEG-DSPE), and poly(hydroxyethyl L-asparagine)-N-succinyl-dioctadecylamine (PHEA-DODASuc), phospholipids such as phosphatidylcholine, phosphatidyl ethanolamine, phosphatidylserine, dipalmitoylphosphatidylcholine, dialkyl-type synthetic surfactants, non-ionic surfactants such as series of Spans (e.g. Span 20, Span 40, Span 60, Span 80) and Tweens (e.g. Tween 20, Tween 60, Tween 80), polyoxyethylene-5-cetyl ether and polyoxyethylene-5-stearyl ether, etc. It will be apparent to one skilled in the art that all of these constituents may be used alone or in combination.

Furthermore, the carrier material may further comprises one or more auxiliary materials and such a material may be selected from one or more of the following: membrane stabilizers, charge modifiers and antioxidants. Examples of auxiliary materials that could be used in conjunction with the method include membrane stabilizers such as sterols (e.g. cholesterol, cholestanol), charge modifiers/inducers such as dicetylphosphate, phosphatidic acid, stearylamine, ganglioside or the like, and antioxidants such as α-tocopherol or the like. Furthermore, the use of ganglioside in accordance with the present invention has been shown to extend the life of the carrier complexes *in vivo.* The proportions of these auxiliary materials to the constituents are not critical and the exact proportion will be dependent on the application of the carrier complex and the intended physiological conditions that the complex will be entering. Preferably, the proportion of membrane stabilizers is about 0.1% to about 30% molar ratio of the membrane components. More preferably, the proportion of membrane stabilizers is about 5% to about 20% molar ratio of the membrane components. The charge modifiers may be about 0.1% to about 50% molar ratio of the membrane components, but may preferably be about 10% to about 30 % molar ratio of the membrane components.

Preferably, the organic solvent is selected from one or more of the following: glycerol, propylene glycol, polyglycerin, polypropylene glycol, maltitol or ethylene glycol. These solvents may be used alone or in combination and do not need to be removed from the final preparation. The proportion of such water-soluble non-volatile organic solvents is preferably about 1 to about 100 parts by weight per part by weight of the carrier complex components and about 0.001 to about 5 parts by weight per part by weight of the aqueous medium.

The aqueous medium may be selected from one or more of the following: water, a physiological saline solution, a buffer solution or an aqueous carbohydrate solution or may be a mixture thereof. Preferably, the pH of the aqueous medium is maintained by a buffer. Such buffers include phosphate-buffered saline (PBS), Tris-HCl buffer, lactate buffer, citrate buffer or the like. For the stability of the carrier complexes the pH should preferably be in the range of 3 to 10. Preferably, the pH will be in the range of 6 to 8. The term "hydration" as used herein means that the components of the carrier complexes are solvated or in free contact with water/organic solvent at the molecular level. Hydration of solid ingredients of the carrier complexes is promoted by stirring, heating and bubbling inert gas through a coarse dispersion of initially non-hydrated molecules.

An active ingredient may be selected from one or more of the following: pharmaceutical compounds, pharmaceutically active salts, polysaccharides, nucleic acids, polypeptides, vitamins and antioxidants. There is no limitation on the number of types of pharmaceutical compounds that can be encapsulated/incorporated into the carrier complexes according to the present invention. For example, the active ingredients may be antitumor drugs such as 5-fluorouracil, methotrexate, doxorubicin, etc., antibiotics such as gentamicin, gramicidin S, etc., polysaccharides such as dextran, etc., nucleic acids such as DNA, RNA and other polynucleotides, vitamins such as vitamin A, etc., antioxidants such as glutathione, etc. and other general drugs such as sodium salicylate, indomethacin, acetaminophen, etc. Indeed, any active substance for the treatment of disease or conditions could be incorporated into the carrier complex. Furthermore, any substance required for research, such as carboxyfluorescein, fluorescein, sulforhodamine and methylene-blue, could also be incorporated into the carrier complex and may be used for the study (such as characterization or uptake) of drug delivery *in vitro* for example. The carrier complexes may be further processed to aid administration of the complexes to the human or animal body and they may additionally be mixed with buffers or other physiologically stable agents to assist administration and/or release of the active ingredients in the complexes.

If the carrier material contains lipids, the temperature in step (b) is more effective to conduct mixing at a temperature not lower than the phase transition temperature (Tc) of the lipid. Alternatively, should the carrier material produce micelles, the ingredients should be used in concentrations above their critical micelle concentration (CMC).

The particle size of the carrier complex can be controlled by the constituents of the carrier material, the speed of the stirring and the rate of inert gas passing through the aqueous medium. Thus when a comparatively mild mixing is performed, the particle sizes of the carrier complexes tend to become relatively bigger. When a high speed mixing and bubbling of inert gas is performed the particle sizes tend to be smaller. Moreover, for the purpose of controlling the particle size distribution of the carrier complexes, it is possible to pass the particles through a filter such as an ultrafiltration unit which may employ a polycarbonate membrane filter for example. Preferably, if an ultrafiltration unit is used in conjunction with the present invention, it generally has a pore size of 400nm or less. When the carrier complex is produced under the standard heat sterilisation conditions (i.e. 120°C for 30 minutes) or when filtered through filters of 400nm pore size or less, there is no need to perform any further sterilisation process. Hence, sterile carrier complexes can be produced using a simple protocol within as little as 30 minutes.

Incorporation of heat-sensitive active ingredients (such as DNA, RNA) to the carrier complexes can be introduced to the complexation zone when the temperature is reduced (e.g. after 30 minutes heating) to an acceptable temperature (e.g. 40°C). In order to assist in the formulation of a stable carrier complex, the carrier material(s) may be selected for a given active ingredient on the basis of its charge. Therefore, the binding, encapsulation or incorporation of these active ingredients to the carrier complexes can be achieved by using oppositely charged carrier complexes, for example, cationic polymer- or lipid-based complexes. These active ingredients can even be incorporated to similarly charged carrier complexes such as anionic polymer- or lipid-based complexes by the mediation of divalent metal cations such as Ca²⁺ or Mg²⁺. High drug entrapment efficiencies can be achieved by use of carrier complex components having a charge opposite to the charge of the active ingredient or drug. When the amount of the active ingredient is not more than 50% on the ion equivalent basis relative to the oppositely charged carrier complex or, more precisely, relative to the charged molecules involved in charging of the carrier complex, a higher active ingredient encapsulation efficiency can be attained.

Preferably, the method is performed in a vessel with a stirring unit disposed in its interior. The vessel may additionally comprise a heating element. Such a vessel may be manufactured from stainless steel or heat resistant glass.

In accordance with another aspect of the present invention, there is an apparatus for producing carrier complexes of an active ingredient disposed within a carrier inert gas supply to its interior, a heating means for heating the interior of the vessel and the vessel further comprising an interior with an undulating cross-section defining a plurality of baffles. The stirring unit may be a magnet stirrer. The heating means may be an element disposed within the interior of the vessel or an element which applies heat to the exterior of the vessel and thus heat is transferred to the interior of the vessel. Additionally, the inert gas may be supplied to the bottom half of the vessel. Furthermore, the inert gas may be supplied at a plurality of points between the baffles. The vessel may have a means to at least partially recycle the inert gas through the vessel. It is possible to prepare the carrier complexes without "bubbling" the inert gas from the bottom of the vessel (in that case to prevent oxidation the top of the vessel can be filled with inert gas during the process) but this will increase the time of the manufacture of the carrier complexes.

Such a vessel may also be connected to a filtration unit (such as an ultrafiltration unit) so as to control the size of the carrier complexes. Preferably, the vessel is manufactured from stainless steel or heat resistant glass, although other materials will be apparent to the skilled addressee. The vessel may also be charged with an aqueous medium. The aqueous medium may comprise a carrier material and a non-volatile water soluble organic solvent and additionally the active ingredient and auxiliary materials such as membrane stabilizers, charge modifiers and antioxidants.

The present invention will now be described by way of example only with reference to and illustrated in the following figures and examples:
Figure 1 illustrates a reaction vessel in accordance with the present invention;
Figure 2 is a perspective view of a reaction vessel in accordance with the present invention;
Figure 3 is a cross sectional view of the reaction vessel in accordance with the present invention, showing the baffles which are formed by the undulating interior;
Figure 4 illustrates the vortices produced by the baffles as shown in Figure 3; and
Figure 5 is a cross sectional view of a reaction vessel in accordance with the present invention.

### EXAMPLE 1

This example illustrates the basic protocol of the method of producing carrier complexes and the apparatus used in the method.

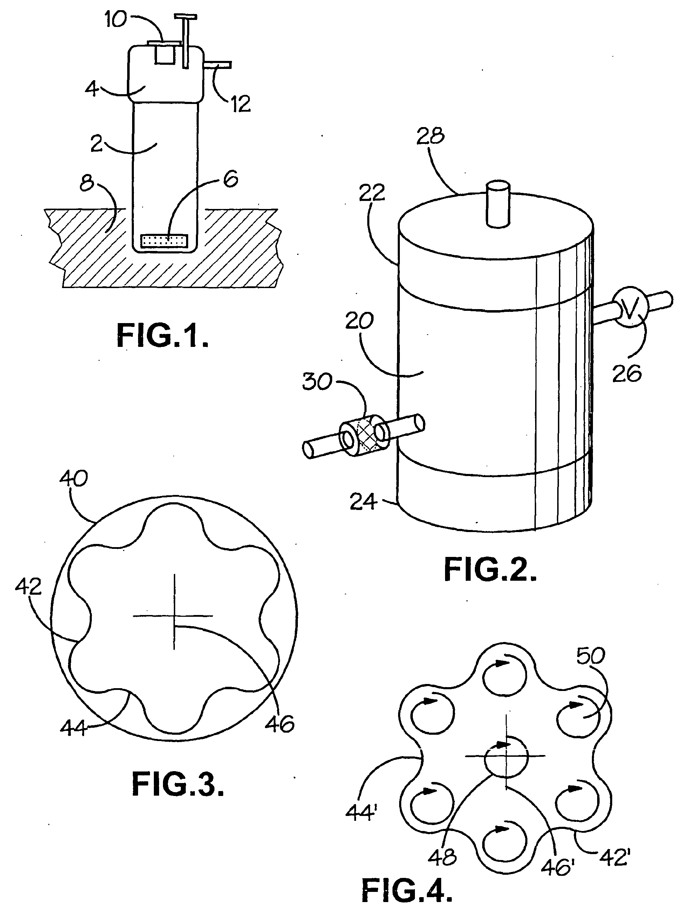

With reference to Figure 1, there is provided an apparatus for producing carrier complexes of an active ingredient disposed within a carrier material having a main tank 2 with a removable cap 4. A stirring magnet 6 is disposed at the base of the tank 2 which rotates about an axis when in operation. A heating unit 8 applies heat to the exterior of the tank 2, which is manufactured from stainless steel heat or heat resistant glass and can therefore conduct heat to the interior of the tank. The tank 2 also has a first valve 10 which permits constituents of the reaction to be placed inside the tank 2 without removal of the cap 4 a second valve 12 (in this case a screw valve) which permits nitrogen to be pumped in or out of the tank 2.

With reference to Figure 2, there is provided an apparatus for producing carrier complexes of an active ingredient disposed within a carrier material, which is similar to the apparatus as shown in Figure 1, as it also has a tank 20 with a removable cap 22 and a heating unit 24. A magnet stirrer is also provided (not shown) and an inlet valve 26 for supplying nitrogen gas to the tank 20. Excess nitrogen or depleted air can be removed by means of the outlet valve 28 and furthermore, this valve can be connected to the nitrogen supply so that excess nitrogen can be recycled. An ultrafiltration unit 30 is also attached to the tank 20 and this allows the carrier complexes to be removed from the tank 20 and the size of the complexes filtered.

Figures 3 and 4 show the interior of an apparatus for producing carrier complexes of an active ingredient disposed within a carrier material, wherein the tank 40 has an undulating interior 42,42' that forms six discrete baffles 44,44' and a cross-shaped stirring bar 46,46' is disposed in the centre of the tank 40. Six inlets (not shown) in between the baffles permit the input of nitrogen into the interior of the tank 40. The action of the stirring bar 46,46' provides for more vigorous stirring and a deeper vortex 48 above the stirring bar 46,46' in addition to creating a number of vortices 50 in between the baffles 44,44'. The vortices 48,50 increase the efficiency and speed of manufacture of carrier complexes with narrow size distribution, which is particularly suited to producing carrier complexes for the pharmaceutical industry.

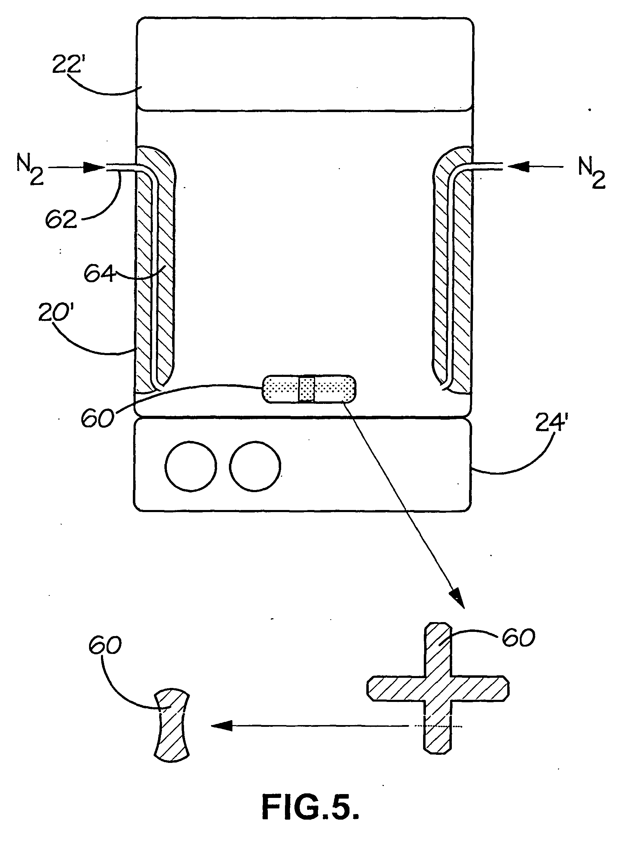

With reference to Figure 5, there is provided an apparatus which is similar in configuration to that shown in Figure 2 as it has a tank 20', a removable cap 22' and a heating means 24' disposed towards the base of the tank. The tank 20' has a number of undulating baffles 64 (similar to those shown in Figure 3 and 4) which are used to deliver the Nitrogen to the base of the interior of the tank by means of a delivery tube 62. A magnetic stirrer 60 is also disposed towards the base of the tank 20' which can rotate at a rate determined by the user. The magnetic stirrer 60 is in a cross configuration with a blade having a sides which are concave is cross section.

In use, the tank 2,20,20',40 is charged with an aqueous medium which contains the carrier material (which may contain the carrier material itself with other materials such as membrane stabilisers or divalent metal cations) and a non-volatile water soluble organic solvent such as glycerol. The stirrer 6,46,48,60 mixes the aqueous medium whilst the heating unit 8,24,24' heats the tank to a temperature between 60°C and 120°C and nitrogen gas is additionally pumped through the medium though valves 12,26,26' or apertures (not shown) in between the baffles 44,44',64. The active ingredient (which may be a pharmaceutical compound for example) can be added to the aqueous medium initially if it is a robust molecule, or introduced to the tank 2,20,20',40 after the initial mixing and the temperature has fallen. The active ingredient may be mixed with the aqueous medium after the initial mixing if the ingredient is for example an enzyme which may denature at high temperatures. To minimise consumption of large amounts of nitrogen gas it can be recirculated in the tank 2,20,20',40 by means of an air pump. The apparatus can also be made in different sizes such as small and medium (laboratory scale) as well as large (industrial scale). Additional units can include a detachable filtration unit 30 and a pump (not shown) or a piston (also not shown) to force the carrier complex suspension through the filter.

### EXAMPLE 2

An experiment was conducted to produce a carrier complex incorporating the active pharmaceutical of paclitaxel ingredient surrounded by a carrier material of a PDLLA-PEG copolymer.

5 mg/ml of PDLLA-PEG copolymer (number average molecular weight of PDLLA: 1866, number average molecular weight of PEG: 3300-4000) was mixed with maltitol (3% v/v) (non-volatile solvent) and paclitaxel (at a concentration of 2 to 10% w/w) and phosphate buffer (pH: 7.4) in the reaction tank (as described above in Example 1) whilst being heated continuously (70 to 120°C), stirred and nitrogen gas bubbling throughout the reaction mixture.

The reaction volume can for example be as small as 5 ml, using the small-sized equipment explained in Example 1, or as large as 1000 1, using the industrial-sized equipment. The carrier complexes are formed within 10 to 30 minutes with high yield, narrow size distribution and high paclitaxel incorporation efficiency. Formation of carrier complexes are attested by microscopic visualisation, size distributions are measured by light scattering and release behaviour studies are performed in buffer or bodily fluids such as plasma/serum, urine, salivary fluid, and bronchoalveolar lavage.

### EXAMPLE 3

A further experiment was conducted to produce a carrier complex incorporating an daunorubicin by a carrier material of a PNIPAAm-*b*-PEG copolymer. 1 mg/ml of PNIPAAm-*b*-PEG copolymer (24300:5000 g/mol) was mixed with glycerin (3% v/v) and daunorubicin (at a concentration of 2 mg/ml) and phosphate buffer (pH: 7.4) in a reaction tank which was heated continuously at a temperature between 70 to 100°C, whilst being stirring and nitrogen gas bubbling through the reaction mixture. The reaction volume as in Example 2, can for example, be as small as 5 ml, using the small-sized equipment explained in Example 1, or as large as 1000 l, using the industrial-sized equipment. Carrier complexes produced using this method are formed within 15 to 60 minutes with a high yield, narrow size distribution and high daunorubicin incorporation efficiency. Again, as with Example 2, formation of carrier complexes are attested by microscopic visualisation, size distributions are measured by light scattering and release behaviour studies are performed in buffer or bodily fluids such as plasma/serum, urine, salivary fluid, and bronchoalveolar lavage.

### EXAMPLE 4

An experiment was conducted to produce a carrier complex incorporating 5-fluorouracil in a DPPC:DCP:CHOL carrier material. DPPC:DCP:CHOL (7:2:1 molar ratio), containing 10mM to 50mM of total lipid, was mixed with propylene glycol (3% v/v) and 5-fluorouracil (at a concentration of 400mM) and phosphate buffered saline (pH: 7.4) in the reaction tank under continuous heating (120°C) conditions, and whilst being stirred continuously and nitrogen bubbled through the reaction mixture. The reaction volume can be between 5 ml and 1000 l. Carrier complexes were formed within 15 to 30 minutes with high yield, narrow size distribution and high 5-fluorouracil incorporation efficiency. Again, formation of carrier complexes are attested by microscopic visualisation, size distributions are measured by light scattering and release behaviour studies are performed in buffer or bodily fluids such as plasma/serum, urine, salivary fluid, and bronchoalveolar lavage.

### EXAMPLE 5

Carrier complexes were formed according to the same protocol as described in Example 4, but with glycerine was substituted in place of propylene glycol with similar results.

### EXAMPLE 6

Carrier complexes were formed according to the same protocol as described in Examples 4 or 5 but with stearylamine (cationic lipid) in place of DCP (anionic lipid) and producing similar results.

### EXAMPLE 7

Carrier complexes were formed according to the same protocol as described in Examples 4 or 5 but with no cholesterol and added at a temperature of 60 to 70°C.

### EXAMPLE 8

Carrier complexes were formed according to the same protocol as described in Examples 4 or 5 but with glutathione in place of 5-fluorouracil.

### EXAMPLE 9

Carrier complexes were formed according to Examples 4 or 5 or 8 but with PEG-PE (PEG: 2000) in place of DPPC:DCP:CHOL at a temperature of 60 to 70°C.

### EXAMPLE 10

Carrier complexes were formed according to Examples 4 or 5 but with no drug. To these 'empty' carrier complexes in the reaction tank, and at a temperature not more than 40°C, plasmid DNA (pcDNA3.1/His B/lacZ, 8578 nucleotides) (Invitrogen) and then calcium chloride (50mM) are added followed by the incubation of the mixture for 15 to 30 minutes. Characterisation of the carrier complex were performed as explained above while transfection efficiency was evaluated by using appropriate cell culture systems such as human respiratory epithelial cells (16HBE14o-).

### EXAMPLE 11

Carrier complexes were formed according to the same protocol as in Examples 4 or 5 or 8 or 10 but with a composition of Span-60:DCP instead of DPPC:DCP:CHOL at a temperature of 40 to 50°C.

### EXAMPLE 12

Carrier complexes were formed according to the same protocol as outlined in Examples 4 or 5 or 8 or 10 but with a composition of Tween 80:DCP in place of DPPC:DCP:CHOL at a temperature of 40 to 50°C.

## Claims

1. A method of producing a carrier complex of an active ingredient disposed within a carrier material, the method comprising the steps of:
(a) providing a complexation zone supplied with an aqueous medium containing the carrier material and a non-volatile water soluble organic solvent;
(b) simultaneously stirring, heating, and passing an inert gas through the aqueous medium;
(c) adding the active ingredient to the aqueous medium and maintaining the complexation zone under conditions of temperature effective to facilitate complexation of the active ingredient by the carrier material; and
(d) recovering from the complexation zone a carrier complex of the active ingredient.

2. A method according to claim 1, wherein step (c) is incorporated into step (a) or (b).

3. A method according to claim 1, wherein step (c) is subjected to simultaneous stirring and the passing of inert gas through the aqueous medium.

4. A method according to any preceding claim, wherein the aqueous medium of step (b) and/or (c) is heated to a temperature in the range of 40°C to 120°C.

5. A method according to any of claims 1 to 3, wherein step (c) is further subjected to a temperature that is less than the temperature of the aqueous medium in step (b).

6. A method as claimed in claim 5, wherein the temperature is in the range of 40°C to 60°C.

7. A method according to any preceding claim, wherein the inert gas comprises nitrogen.

8. A method as claimed in any preceding claim, wherein the carrier complex comprises a microsphere, a nanosphere, a liposome, a vesicular phospholipid gel, a archaeosome, a niosome, a micelle or a nanoparticle.

9. A method as claimed in any preceding claim, wherein the carrier material is selected from one or more of the following:
a copolymer, a synthetic polymer-lipid conjugate, a phospholipid or a non-ionic surfactant.

10. A method as claimed in any preceding claim, wherein the carrier material further contains one or more auxiliary materials selected from one or more of the following:
membrane stabilizers, charge modifiers and antioxidants.

11. A method as claimed in claim 10, wherein the proportion of membrane stabilizers is in the range of 0.1 - 30% molar ratio of the carrier material.

12. A method as claimed in either claim 10 or 11, wherein the proportion of charge modifiers is in the range of 0.1 - 50% molar ration of the carrier material.

13. A method as claimed in any preceding claim, wherein the organic solvent is selected from one or more of the following:
glycerol, propylene glycol, polyglycerin, polypropylene glycol, maltitol or ethylene glycol.

14. A method as claimed in any preceding claim, wherein the organic solvent is in the range of 1 to 100 parts by weight per part by weight of the carrier complex components and 0.001 to about 5 parts by weight per part by weight of the aqueous medium.

15. A method as claimed in any preceding claim, wherein the aqueous medium is selected from one or more of the following:
water, a physiological saline solution, a buffer solution or an aqueous carbohydrate solution.

16. A method as claimed in any preceding claim, wherein the pH of the aqueous medium is maintained in the range of 3 to 10.

17. A method as claimed in any preceding claim, wherein the active ingredient is selected from one or more of the following:
pharmaceutical compounds, pharmaceutically active salts, polysaccharides, nucleic acids, polypeptides, vitamins and antioxidants.

18. A method as claimed in any preceding claim, wherein if the carrier material contains lipids, the temperature in step (b) is not lower than the phase transition temperature of the lipid.

19. A method as claimed in any preceding claim, wherein the method further comprises the step of passing the carrier complex through an ultrafiltration unit to control the particle size of the carrier complexes.

20. A method as claimed in claim 19, wherein the ultrafiltration has a pore size of 400nm or less.

21. A method as claimed in any preceding claim, wherein divalent metal cations are used to mediate the binding of carrier material(s) and the active ingredient.

22. An apparatus for producing a carrier complex of an active ingredient disposed within a carrier material, the apparatus comprising a vessel with a stirring unit disposed in its interior, a inert gas supply to its interior, a heating means for heating the interior of the vessel and the vessel further comprising an interior with an undulating cross-section defining a plurality of baffles.

23. An apparatus as claimed in claim 22, wherein the inert gas is supplied to the bottom half of the vessel.

24. An apparatus as claimed in either claims 22 or 23, wherein the inert gas is supplied at a plurality of points between the baffles.

25. An apparatus as claimed in any of claims 22 to 24, wherein the vessel is connected to a filtration unit so as to control the size of the carrier complexes.

26. A carrier complex of an active ingredient disposed within a carrier material as made according to the method as claimed in claims 1 to 21, in an apparatus as claimed in claims 22 to 25.

## Patentansprüche

1. Verfahren zur Herstellung eines Trägerkomplexes eines in einem Trägermaterial befindlichen Wirkstoffs, wobei das Verfahren die folgenden Schritte beinhaltet:
(a) Bereitstellen einer Komplexierungszone, die mit einem wässrigen Medium versorgt wird, das das Trägermaterial und ein nicht flüchtiges, wasserlösliches organisches Lösungsmittel enthält;
(b) gleichzeitig Rühren, Erhitzen und Leiten eines Inertgases durch das wässrige Medium;
(c) Zugeben des Wirkstoffs zu dem wässrigen Medium und Halten der Komplexierungszone unter Temperaturbedingungen, die die Komplexierung des Wirkstoffs durch das Trägermaterial erleichtern; und
(d) Gewinnen eines Trägerkomplexes des Wirkstoffs von der Komplexierungszone.

2. Verfahren nach Anspruch 1, wobei Schritt (c) in Schritt (a) oder (b) einbezogen wird.

3. Verfahren nach Anspruch 1, wobei Schritt (c) bei gleichzeitigem Rühren und Leiten von Inertgas durch das wässrige Medium erfolgt.

4. Verfahren nach einem der vorherigen Ansprüche, wobei das wässrige Medium aus Schritt (b) und/oder (c) auf eine Temperatur im Bereich von 40°C bis 120°C erhitzt wird.

5. Verfahren nach einem der Ansprüche 1 bis 3, wobei Schritt (c) ferner bei einer Temperatur unter der Temperatur des wässrigen Mediums in Schritt (b) erfolgt.

6. Verfahren nach Anspruch 5, wobei die Temperatur im Bereich von 40°C bis 60°C liegt.

7. Verfahren nach einem der vorherigen Ansprüche, wobei das Inertgas Stickstoff umfasst.

8. Verfahren nach einem der vorherigen Ansprüche, wobei der Trägerkomplex eine Mikrosphäre, eine Nanosphäre, ein Liposom, ein vesikuläres Phospholipidgel, ein Archaeosom, ein Niosom, eine Mizelle oder einen Nanopartikel umfasst.

9. Verfahren nach einem der vorherigen Ansprüche, wobei das Trägermaterial ausgewählt wird aus einem oder mehreren der Folgenden:
einem Copolymer, einem synthetischen Polymer-Lipid-Konjugat, einem Phospholipid oder einem nichtionischen Tensid.

10. Verfahren nach einem der vorherigen Ansprüche, wobei das Trägermaterial ferner ein oder mehrere Hilfsmaterialien enthält, ausgewählt aus einem oder mehreren der Folgenden:
Membranstabilisatoren, Ladungsmodifizierer und Antioxydationsmittel.

11. Verfahren nach Anspruch 10, wobei der Anteil der Membranstabilisatoren in einem Molverhältnisbereich von 0,1 bis 30 % zum Trägermaterial liegt.

12. Verfahren nach Anspruch 10 oder 11, wobei der Anteil der Ladungsmodifizierer in einem Molverhältnisbereich von 0,1 bis 50 % zum Trägermaterial liegt.

13. Verfahren nach einem der vorherigen Ansprüche, wobei das organische Lösungsmittel ausgewählt wird aus einem oder mehreren der Folgenden:
Glycerol, Propylenglykol, Polyglycerin, Polypropylenglykol, Maltitol oder Ethylenglykol.

14. Verfahren nach einem der vorherigen Ansprüche, wobei das organische Lösungsmittel im Bereich von 1 bis 100 Gewichtsteilen je Gewichtsteil der Trägerkomplexlcomponenten und 0,001 bis etwa 5 Gewichtsteilen je Gewichtsteil des wässrigen Mediums liegt.

15. Verfahren nach einem der vorherigen Ansprüche, wobei das wässrige Medium ausgewählt wird aus einem oder mehreren der Folgenden:
Wasser, einer physiologischen Kochsalzlösung, einer Pufferlösung oder einer wässrigen Kohlenhydratlösung.

16. Verfahren nach einem der vorherigen Ansprüche, wobei der pH-Wert des wässrigen Mediums im Bereich von 3 bis 10 gehalten wird.

17. Verfahren nach einem der vorherigen Ansprüche, wobei der Wirkstoff ausgewählt wird aus einem oder mehreren der Folgenden:
pharmazeutischen Verbindungen, pharmazeutisch aktiven Salzen, Polysacchariden, Nucleinsäuren, Polypeptiden, Vitaminen und Antioxydationsmitteln.

18. Verfahren nach einem der vorherigen Ansprüche, wobei, wenn das Trägermaterial Lipide enthält, die Temperatur in Schritt (b) nicht niedriger als die Phasenübergangstemperatur des Lipids ist.

19. Verfahren nach einem der vorherigen Ansprüche, wobei das Verfahren ferner den Schritt des Leitens des Trägerkomplexes durch eine Ultrafiltrationseinheit zum Regulieren der Partikelgröße der Trägerkomplexe beinhaltet.

20. Verfahren nach Anspruch 19, wobei die Ultrafiltration eine Porengröße von 400 nm oder weniger hat.

21. Verfahren nach einem der vorherigen Ansprüche, wobei bivalente Metallkationen zum Vermitteln der Bindung des/der Trägermaterials/-ien und des Wirkstoffs verwendet werden.

22. Vorrichtung zur Herstellung eines Trägerkomplexes eines in einem Trägermaterial befindlichen Wirkstoffs, wobei die Vorrichtung Folgendes umfasst: ein Gefäß mit einer Rühreinheit im Innenbereich, eine Inertgaszufuhr zu seinem Innenbereich, ein Erhitzungsmittel zum Erhitzen des Innenbereichs des Gefäßes, wobei das Gefäß ferner einen Innenbereich mit einem wellenförmigen Querschnitt umfasst, der eine Mehrzahl von Ablenkflächen definiert.

23. Vorrichtung nach Anspruch 22, wobei das Inertgas zur unteren Hälfte des Gefäßes geführt wird.

24. Vorrichtung nach Anspruch 22 oder 23, wobei das Inertgas an mehreren Stellen zwischen den Ablenkflächen zugeführt wird.

25. Vorrichtung nach einem der Ansprüche 22 bis 24, wobei das Gefäß mit einer Filtrationseinheit verbunden ist, um die Größe der Trägerlcomplexe zu regulieren.

26. Trägerkomplex eines in einem Trägermaterial befindlichen Wirkstoffs, hergestellt mit dem Verfahren nach den Ansprüchen 1 bis 21 in einer Vorrichtung nach den Ansprüchen 22 bis 25.

## Revendications

1. Méthode de production d'un complexe véhicule d'un ingrédient actif disposé au sein d'un matériau véhicule, la méthode comprenant les étapes :
(a) de fourniture d'une zone de complexation fournie avec un milieu aqueux contenant le matériau véhicule et un solvant organique hydrosoluble non volatile;
(b) de simultanément agiter, chauffer et passer un gaz inerte à travers le milieu aqueux ;
(c) d'ajouter l'ingrédient actif au milieu aqueux et de maintenir la zone de complexation dans des conditions de températures efficaces pour faciliter la complexation de l'ingrédient actif par le matériau véhicule ; et
(d) de récupération à partir de la zone de complexation, d'un complexe véhicule de l'ingrédient actif.

2. Méthode selon la revendication 1, dans laquelle l'étape (c) est incorporée dans l'étape (a) ou (b).

3. Méthode selon la revendication 1, dans laquelle l'étape (c) est soumise à l'agitation et au passage simultanés de gaz inerte à travers le milieu aqueux.

4. Méthode selon l'une quelconque des revendications précédentes, dans laquelle le milieu aqueux de l'étape (b) et/ou (c) est chauffé jusqu'à une température dans la plage allant de 40°C à 120°C.

5. Méthode selon l'une quelconque des revendications 1 à 3, dans laquelle l'étape (c) est soumise en outre à une température qui est inférieure à la température du milieu aqueux dans l'étape (b).

6. Méthode selon la revendication 5, dans laquelle la température se trouve dans la plage allant de 40°C à 60°C.

7. Méthode selon l'une quelconque des revendications précédentes, dans laquelle le gaz inerte comprend de l'azote.

8. Méthode selon l'une quelconque des revendications précédentes, dans laquelle le complexe véhicule comprend une microsphère, une nanosphère, un liposome, un gel phospholipidique vésiculaire, un archéosome, un niosome, une micelle ou une nanoparticule.

9. Méthode selon l'une quelconque des revendications précédentes, dans laquelle le matériau véhicule est choisi parmi un ou plusieurs dans la liste suivante :
un copolymère, un conjugué lipide-polymère synthétique, un phospholipide ou un agent tensioactif non ionique.

10. Méthode selon l'une quelconque des revendications précédentes, dans laquelle le matériau véhicule contient en outre un ou plusieurs matériaux auxiliaires choisis parmi un ou plusieurs dans la liste suivante :
des stabilisants membranaires, des modificateurs de charge et des antioxydants.

11. Méthode selon la revendication 10, dans laquelle la proportion de stabilisants membranaires se trouve dans la plage de 0,1-30% du rapport molaire du matériau véhicule.

12. Méthode selon la revendication 10 ou bien 11, dans laquelle la proportion de modificateurs de charge se trouve dans la plage de 0,1-50% du rapport molaire du matériau véhicule.

13. Méthode selon l'une quelconque des revendications précédentes, dans laquelle le solvant organique est choisi parmi un ou plusieurs dans la liste suivante :
le glycérol, le propylène glycol, la polyglycérine, le polypropylène glycol, le maltitol ou l'éthylène glycol.

14. Méthode selon l'une quelconque des revendications précédentes, dans laquelle le solvant organique se trouve dans la plage allant de 1 à 100 parties en poids par partie en poids des composants du complexe véhicule et de 0,001 à environ 5 parties en poids par partie en poids du milieu aqueux.

15. Méthode selon l'une quelconque des revendications précédentes, dans laquelle le milieu aqueux est choisi parmi un ou plusieurs dans la liste suivante :
l'eau, une solution saline physiologique, une solution tampon ou une solution glucidique aqueuse.

16. Méthode selon l'une quelconque des revendications précédentes, dans laquelle le pH du milieu aqueux est maintenu dans la plage allant de 3 à 10.

17. Méthode selon l'une quelconque des revendications précédentes, dans laquelle l'ingrédient actif est choisi parmi un ou plusieurs dans la liste suivante :
des composés pharmaceutiques, des sels pharmaceutiquement actifs, des polysaccharides, des acides nucléiques, des polypeptides, des vitamines et des antioxydants.

18. Méthode selon l'une quelconque des revendications précédentes, dans laquelle, si le matériau véhicule contient des lipides, la température dans l'étape (b) n'est pas inférieure à la température de transition de phase du lipide.

19. Méthode selon l'une quelconque des revendications précédentes, dans laquelle la méthode comprend en outre l'étape de passer le complexe véhicule à travers un module d'ultrafiltration afin de contrôler la taille de particules des complexes véhicules.

20. Méthode selon la revendication 19, dans laquelle l'ultrafiltration a une taille de pores de 400 nm ou moins.

21. Méthode selon l'une quelconque des revendications précédentes, dans laquelle des cations métalliques divalents sont utilisés pour médier la fixation du ou des matériaux véhicules et de l'ingrédient actif.

22. Appareil de production d'un complexe véhicule d'un ingrédient actif disposé au sein d'un matériau véhicule, l'appareil comprenant un récipient ayant un module d'agitation disposée à l'intérieur, une alimentation en gaz inerte vers son intérieur, un moyen de chauffage pour chauffer l'intérieur du récipient, le récipient comprenant en outre un intérieur ayant une section ondulée définissant une pluralité de chicanes.

23. Appareil selon la revendication 22, dans lequel le gaz inerte est alimenté à la moitié inférieure du récipient.

24. Appareil selon la revendication 22 ou bien 23, dans lequel le gaz inerte est alimenté au niveau d'une pluralité de points entre les chicanes.

25. Appareil selon l'une quelconque des revendications 22 à 24, dans lequel le récipient est relié à un module de filtration de façon à contrôler la taille des complexes véhicules.

26. Complexe véhicule d'un ingrédient actif disposé au sein d'un matériau véhicule, tel que produit selon la méthode selon les revendications 1 à 21, dans un appareil selon les revendications 22 à 25.
